(19)

Europäisches Patentamt
European Patent Office
Office européen des brevets

(11) **EP 3 225 213 A1**

(12) **EUROPEAN PATENT APPLICATION**

(43) Date of publication:
04.10.2017 Bulletin 2017/40

(51) Int Cl.:
*A61F 2/38* (2006.01)    *A61F 2/30* (2006.01)

(21) Application number: 16185377.5

(22) Date of filing: 31.08.2011

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 07.09.2010 GB 201014824
13.09.2010 US 382163 P

(62) Document number(s) of the earlier application(s) in
accordance with Art. 76 EPC:
11760533.7 / 2 613 740

(71) Applicants:
- **Biomet UK Limited**
  **Bridgend**
  **South Wales CF31 3XA (GB)**
- **O'Connor, John**
  **Oxford, Oxfordshire OX38 JN (GB)**
- **Dodd, Chris Alexander**
  **Oxford, Oxfordshire OX26  6RR (GB)**
- **Hunsley, Colin**
  **Hants RG22 5LZ (GB)**
- **Murray, David Wycliffe**
  **Oxford, Oxfordshire OX44 9HB (GB)**

(72) Inventors:
- **O'Connor, John**
  **Oxford, Oxfordshire OX38 JN (GB)**
- **Dodd, Chris Alexander**
  **Oxford, Oxfordshire OX26 6RR (GB)**
- **Murray, David Wycliffe**
  **Oxford, Oxfordshire OX44 9HB (GB)**
- **Goodfellow, John**
  **deceased (GB)**

(74) Representative: **Mays, Julie et al**
**Venner Shipley LLP**
**200 Aldersgate**
**London, EC1A 4HD (GB)**

Remarks:
•This application was filed on 23.08.2016 as a
divisional application to the application mentioned
under INID code 62.
•Claims 16-33 are deemed to be abandoned due to
non-payment of the claims fees (Rule 45(3) EPC).

(54) **UNICONDYLAR MENISCAL BEARING KNEE REPLACEMENT**

(57) A kit of parts for use in unicondylar meniscal bearing knee replacement comprises a plurality of meniscal bearings, each meniscal bearing comprising a body defining a dished first bearing surface on one side thereof and a second surface on an opposing side of the body. Each meniscal bearing has an entrapment between 3.2mm and 3.8mm. Meniscal bearings and methods of performing unicondylar meniscal bearing replacements are also described.

Fig. 4

**Description**

**FIELD OF THE INVENTION**

The present invention relates to a kit of parts for use in unicondylar meniscal bearing knee replacement, a meniscal bearing for use in unicondylar meniscal bearing knee replacement and a method of performing unicondylar meniscal bearing knee replacement.

**BACKGROUND OF THE INVENTION**

The knee comprises three inter-dependent joints in three separate compartments, all surrounded by a fibrous capsule covered by the skin. The medial tibio-femoral joint involves contact between the thigh bone (the femur) and the leg bone (the tibia) on the inside of the lower limb. The lateral tibio-femoral joint involves contact between the femur and the tibia on the outside of the lower limb. The patello-femoral joint involves contact between the femur and the knee cap (the patella) on the front of the lower limb.

The front of the lower (distal) end of the femur comprises an anticlastic flanged groove, convex in the sagittal plane, transversely concave, providing a track for the patella. The back of the distal femur divides into two separate near-spherical convex condyles making contact with the tibia. The upper surface of the tibia is like a plateau which is slightly dished on the medial side for contact with the medial femoral condyle forming the medial tibio-femoral joint and slightly convex on the lateral side for contact with the lateral femoral condyle forming the lateral tibio-femoral joint with a protrusion (the tibial eminence) running from front to back between the joints.

The articulating surfaces in each joint are covered with thin layers of a tough protective layer, called cartilage, and are lubricated by synovial fluid secreted from a membrane on the inner surface of the fibrous capsule surrounding the knee. The surfaces of the tibio-femoral joints are further separated-by the menisci, semi-circular semi-lunar collagen bundles oriented circumferentially. Each bundle is securely attached at each end to the tibia and loosely to the peripheral capsule. The menisci form closely-fitting mobile sockets for the femoral condyles bringing the dissimilar surfaces of the femur and tibia into closer conformity while allowing some antero-posterior translation of the femoral condyles on the tibia.

The bones are held together actively by muscles with their tendons which span the joints and passively by ligaments and the joint capsule. The ligaments comprise bundles of collagen fibres running mainly longitudinally. The collateral ligaments arise on the external surfaces of the medial and lateral condyles. The medial collateral ligament inserts into the external medial surface of the proximal tibia. The lateral collateral ligament inserts into the proximal surface of the fibula. The medial collateral ligament is a much larger and stiffer structure than the lateral collateral ligament. The cruciate ligaments arise from the internal surfaces of the femoral condyles and insert into the tibial eminence.

The ligaments and the bones together form a mechanism which controls a complex pattern of movement of the bones on each other. In the unloaded state, flexion of the knee to 130° about a transverse axis is accompanied by approximately 25° rotation about the axis of the tibia (axial rotation). These movements are accommodated by mainly antero-posterior translations of the tibio-femoral contact areas so that the bones roll as well as slide on each other and the patella slides over the anterior femur. Additionally, the femoral condyles can spin about the axis of the tibia. The joint also allows approximately 5° of rotation about an anteroposterior axis (abduction-adduction). Under load, the ligaments stretch and the articular surfaces indent, significantly modifying the relationship between flexion, axial rotation and abduction-adduction and between flexion and contact area translations. Movements at the knee are therefore load- and activity-dependent.

Damage to the articular surfaces or to the ligaments changes the patterns of movement of the bones on each other and the response of the joint to load. Osteoarthritis follows from failure of the cartilage in one or other of the three joints, leading to bone-on-bone contact and the onset of pain. Frequently, osteoarthritis first manifests itself in the medial compartment, while the ligaments remain intact. The disease can remain confined to the medial compartment until the anterior cruciate ligament fails and the disease then spreads to the other two compartments. No drug based treatment has been found which halts or reverses these processes.

Total knee replacement is the most common surgical treatment for osteoarthritis, involving replacement of the articular surfaces of all three compartments and sacrifice of some of the ligaments. Partial knee replacement involves replacement of the articular surfaces in only one compartment, leaving intact the surfaces of the other two compartments and all of the ligaments. Partial knee replacement can act prophylactically, reducing the rate of development of the disease in the other compartments. Partial knee replacement is surgically more demanding and is therefore not always used when it is indicated.

To implant the prosthetic components of a knee replacement, sufficient sections of bone have to be removed from the surfaces of the tibia and the femur. The component parts of the prosthesis are then fitted accurately replacing the material removed by the surgeon.

Mobile bearing arthroplasty uses metal components fixed to the tibia and the femur with an intervening plastic bearing, an analogue of the natural meniscus, interposed therebetween. The bearing provides a mobile socket to bring the femoral component into conformity with the tibial component. The bearing has a concave socket on its upper surface for contact with the femoral component and a flatter lower surface for contact with the tibial component. The metal components are fixed to the bones so as to leave a constant minimum gap between them when the knee is flexed and extended. The most appropriate thickness of bearing is then chosen to fill that gap.

On implantation, the bearing is pushed between the metal femoral and tibial components against the resistance of stretching ligaments. This requires the thickest part of the posterior portion of the bearing to fit through the minimum gap between the round femoral component and the flatter tibial component. The ligament stretch required is the difference between the maximum thickness of the posterior end of the bearing and the minimum thickness of the bearing. This difference is known as the entrapment of the bearing.

In one version of the prior art, there are five sizes of bearing, from extra small to extra large, with dimensions other than thickness which vary parametrically with the radius of the spherical surface of the femoral component. The entrapment varies from about 3mm in the extra-small to about 4mm in the extra-large. The range of sizes is necessary to fit both small patients and large patients.

A complication of mobile bearing arthroplasty can be slack ligaments. As a result, the surgeon naturally seeks to use the thickest possible bearing to avoid slackness. Care has to be taken not to overstuff the joint, whereby a bearing that is too thick is selected. This leads to pain and failure of the components and, in the case of a partial knee replacement, degeneration of the preserved compartments.

It has been appreciated that post-operative pain is most commonly experienced after implantation of the smaller components. However, pain can also occur in larger bearings, as they require significant force for implantation, provided by the surgeon's thumbs.

It has been appreciated that some patients continue to have pain after a mobile bearing partial knee replacement. There is need for a bearing design with more entrapment for the smaller bearings and less entrapment for the larger bearings which may prevent overstuffing of the joint.

## SUMMARY OF THE INVENTION

According to a first aspect the invention comprises a kit of parts for use in unicondylar meniscal bearing knee replacement, comprising a plurality of meniscal bearings, each meniscal bearing comprising a body defining a dished first bearing surface on one side thereof and a second surface on an opposing side of the body, in which each meniscal bearing has an entrapment, the entrapment of each meniscal bearing being between 3.2mm and 3.8mm.

Previously, it has been thought necessary to vary the entrapment dependent strongly upon the size of the patient and so the size of the medial bearing used. We have appreciated that, for smaller patients and bearings, prior art entrapments, which were generally smaller than the range discussed, did not sufficiently prevent dislocation of the knee joint. Furthermore, the relatively low entrapment meant that a surgeon could be tempted to implant a thicker bearing than is needed for very small patients. For larger patients and hence bearings, the reduction in entrapment from prior art bearings will make the bearing easier to fit.

Where the meniscal bearing has an anterior end and a posterior end, the entrapment may be defined as the difference in thickness between smaller of the maximum thicknesses between the first and second surfaces at the anterior and posterior end on the one part and the minimum thickness between the first and second surfaces on the other part.

The entrapment of the bearings may all be approximately the same, and may be 3.5mm, with a typical tolerance of $\pm$ 0.1mm.

Each meniscal bearing may have a length from an anterior end to a posterior end. The meniscal bearings may have a plurality of different lengths, which may depend upon a radius of curvature of the first surface. The length of at least one of the meniscal bearings may be at least 39mm, or at least 39.4mm. Alternatively, the length of at least one of the meniscal bearings may be at least 36.8mm. The length of at least one of the meniscal bearings may be less than 34.2mm, 34mm, 31.6mm or 29.5mm. Previously, kits of meniscal bearings would include at least one bearing of these sizes with an entrapment outside the range referred to above.

The meniscal bearings may have a plurality of different minimum thicknesses between the first and second surfaces. The meniscal bearings of a given length may have a plurality of different minimum thicknesses, but the same, or approximately the same, entrapment.

The kit may comprise meniscal bearings having a plurality, or all, of the lengths selected from the set comprising 29.2mm (extra small), 31.6mm (small), 34.2mm (medium), 36.8mm (large) and 39.5mm (extra large). The tolerances on the measurements may be up to 0.5mm. For each of the lengths of the set, there may be meniscal bearings having plurality, or all, of minimum thicknesses selected from the group comprising 7.0mm, 8.0mm, 9.0mm, 10.0mm and 11.0mm. The tolerance of the thicknesses may be 0.25mm.

As said before, the entrapment of all of these bearings will be in the range 3.2mm to 3.8mm, which is a considerable narrowing of the range of entrapments compared with prior art bearings. Compared to the prior art, the smaller bearings have an increased entrapment and the larger bearings have a decreased entrapment. For a smaller patient with a limited value of possible ligament stretch, the surgeon may be required to implant a thinner bearing compared to the prior art, resulting in slacker ligaments and less postoperative pain but without significantly increasing the risk of dislocation. In the larger patients with a limited value of ligament stretch, a bearing of the correct minimum thickness should be easier to implant compared to the prior art.

The meniscal bearings may be trial bearings for use in fitting a prosthesis, or implantable bearings for use with the prosthesis. The kit may comprise a set of trial bearings and a set of implantable bearings, each implantable bearing corresponding to a trial bearing.

The correspondence between trial and implantable bearings may be such that the surgeon fits the thickest grade trial bearing they can into the gap between the tibial and femoral components of a patient's knee, and then removes the trial bearing and selects the implantable bearing to be one grade thinner than the thickest trial bearing that fitted in the gap, and implants the selected bearing.

It will be appreciated that compared to the prior art, the smaller sets of implantable and trial bearings may have a greater entrapment and a thicker posterior end region (typically up to 0.5mm thicker for the equivalent grade of a size range).

This should reduce the chance of dislocation of the bearing in use, and reduce the chance of overstuffing the joint by requiring the surgeon to choose a bearing of reduced minimum thickness compared to the prior art.

In some embodiments the meniscal bearings are symmetrical about either or both of a coronal and a sagittal plane; as such, they may be able to be inserted into the gap between the tibial and femoral prosthetic components either way around. This reduces the number of components required for manufacture, as the bearings will no longer be handed.

The kit may comprise at least one femoral prosthetic component having a spherical articular surface having a radius of curvature, the kit comprising meniscal bearings in which the first surface has the same radius of curvature. The kit may also comprise at least one tibial prosthetic component.

At least some embodiments of the present invention provide a bearing with a substantially conforming contact area between the articulating surfaces of the femoral component and the tibial component throughout extension and flexion. The bearing also provides an increase in and a substantially constant articulating contact surface area from extension through flexion which reduces overall stress and wear in the articulating contact area which provides femoral rollback relative to the tibial component.

Each meniscal bearing may comprise a protrusion at a posterior end thereof, an anterior thereof or both. This effectively increases a horizontal entrapment of the bearing.

Without a protrusion on the posterior end, the bearing could be easier to implant if it were slid around the femoral component rather than along the tibial component. This would increase the likelihood of overstuffing for a given entrapment. The posterior protrusion makes it more difficult to implant the bearing in this manner, thereby reducing the risk of overstuffing.

The protrusion may be semi-circular or any other convenient shape. The extent of the protrusion may be such so as not to press against the vital vessels passing across the back of the joint in use and can be designed to make implantation along the tibial component and the femoral component equally difficult.

A protrusion on the anterior surface of the bearing will decrease the likelihood of the bearing dislocating in the posterior direction. Likewise, a protrusion added to the posterior end of the bearing will decrease the likelihood of the bearing dislocating in the anterior direction.

The protrusion may be of even depth along the end to which it is added. Alternatively, the protrusion may have a reduced depth at the upper end of the surface compared with the lower end, giving a sloped edge. This may reduce the chance of the upper surface of the bearing coming into contact with the bone surrounding the femoral component.

According to a second aspect of the invention, there is provided a meniscal bearing for use in a unicondylar meniscal) bearing knee replacement, comprising a body defining a dished first bearing surface on one side thereof and a second surface on an opposing side of the body, the meniscal bearing having a length from an anterior end to a posterior end being greater than 39mm or less than 34.2mm, and having an entrapment being the maximum difference in thickness between the first and second surfaces of between 3.2mm and 3.8mm. Preferably, the length is greater than 39.4mm, or less than 34mm or 31.6mm.

The bearing may have any of the optional features discussed with respect to the meniscal bearing included in the kit of the first aspect of the invention.

According to third aspect of the invention, there is provided a method of performing a unicondylar meniscal bearing knee replacement, the method comprising the implantation of a femoral component into the femur of a patient, the implantation of a tibial component into the tibia of the patient, the selection of a meniscal bearing from the kit of the first aspect of the invention, or of a meniscal bearing according to the second aspect of the invention, and the insertion of the meniscal bearing between the femoral component and the tibial component.

In one embodiment, the method comprises inserting the bearing at an angle to an particular surface of tibial component so that the first surface of the meniscal. bearing substantially contacts a bearing surface of the femoral component throughout insertion of the meniscal bearing. This may reduce the distraction of the femoral component that occurs during the implanting of the meniscal bearing compared to if the bearing is inserted with the second surface of the bearing substantially contacting the tibial tray. Reducing the distraction of the femoral component reduces the amount of ligament stretch required for implantation. For a given limit of ligament stretch and minimum bearing thickness, a bearing with a larger entrapment can be inserted into the gap without reaching the limit of ligament stretch, resulting in a better fitting bearing.

## BRIEF DESCRIPTION OF DRAWINGS

The invention will now be described by way of example only, with reference to the accompanying drawings, of which:-

**Figure 1** shows a medial unicondylar arthroplasty;

**Figures 2a and 2b** show schematically a meniscal bearing of the prosthesis of Figure 1;

**Figure 2c** shows schematically a prior art anatomically-shaped meniscal bearing

**Figure 2d** shows schematically a prior art meniscal bearing;

**Figures 3a and 3b** show a trial fitting of a trial meniscal bearing in a procedure to implant the unicondylar implant of Figure 1;

**Figure 4** shows a meniscal bearing and geometry for illustrating the relationship between radius of curvature R, and some geometrical features of the bearing; and

**Figure 5** shows schematically a unicondylar arthroplasty;

**Figure 6a** shows a meniscal bearing implantation along the tibial component;

**Figure 6b** shows a meniscal bearing implantation along the femoral component;

**Figure 7** shows schematically another embodiment of a unicondylar arthroplasty; and

**Figure 8** shows another embodiment of a meniscal bearing implantation along the femoral component.

## DESCRIPTION OF EMBODIMENTS

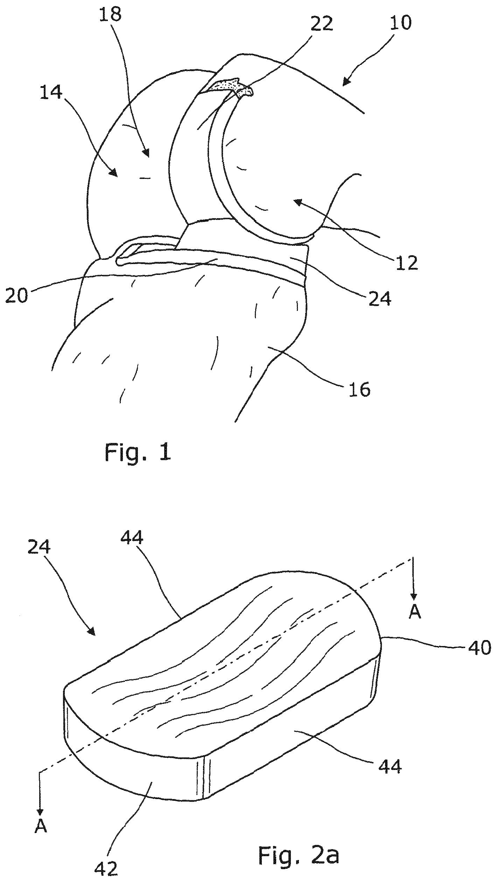

Figure 1 shows a right leg femur 10 having a medial condyle 12 and a lateral condyle 14, a tibia 16, and a medial unicondylar implant 18. The implant has three components: a metal tibial plate, or component, 20, a metal femoral component 22 and a plastics material (e.g. Ultra high Molecular weight polyethylene, UHMWPE) meniscal bearing 24. The materials named are those commonly used but they are not essential.

The bottom, second, surface of the meniscal bearing 24 is flat or generally flat, whereas the upper, first, surface is a dished, concave, surface which surrounds the domed surface of the femoral component 22.

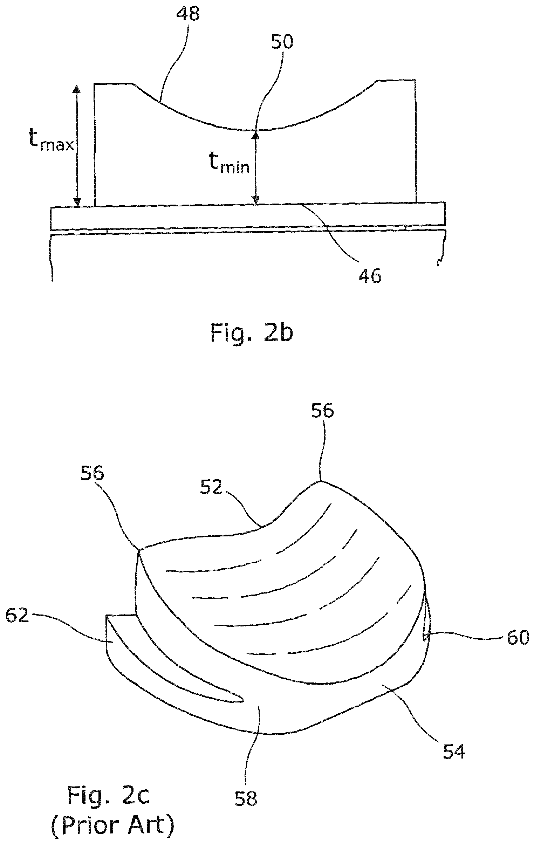

Figures 2a and 2b show schematically the meniscal bearing of Figure 1. In this embodiment, the meniscal bearing 24 has a posterior end 40, an anterior end 42 and two generally parallel sides 44. The meniscal bearing 24 has a flat base 46 forming the second surface and a central dished upper portion 48 forming the first surface. The thickness of this bearing (reference $t_{max}$ in Figure 2b) may not be the same at the anterior and posterior ends. The base surface 50 of the dished portion 48 is a distance $t_{min}$ above the flat base 46. The surface of the dished portion is generally part of a spherical surface. The entrapment of the bearing is $t_{max}$-$t_{min}$.

Figure 2c shows schematically an alternative design of meniscal bearing which has an anatomical shape. While the lateral side 52 and the medial side 54 are still generally parallel, the medial side 54 is shorter than the lateral side 52, and the medial corners 58 and 60 are of larger radii than those of lateral corners 56, which generally have equal radii. Anteromedial corner 58 may have a larger radius than posteromedial corner 60 to minimise bearing overhang in extension. The bearing also includes protrusions 62 extending from the lower portion of lateral corners 56 to the lower portion of medial corners 58 and 60. The anatomical shape reduces the likelihood of the bearing rotating on its flat base around a vertical axis, as the additional material comes into contact with the tibial wall.

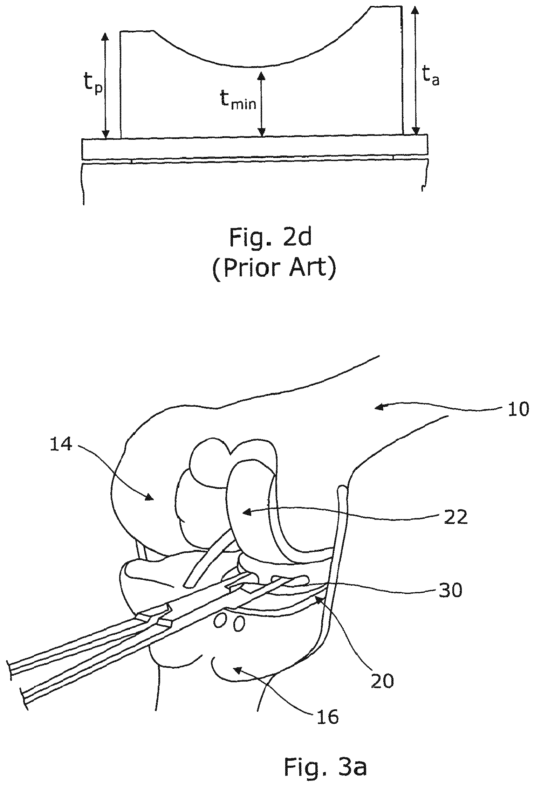

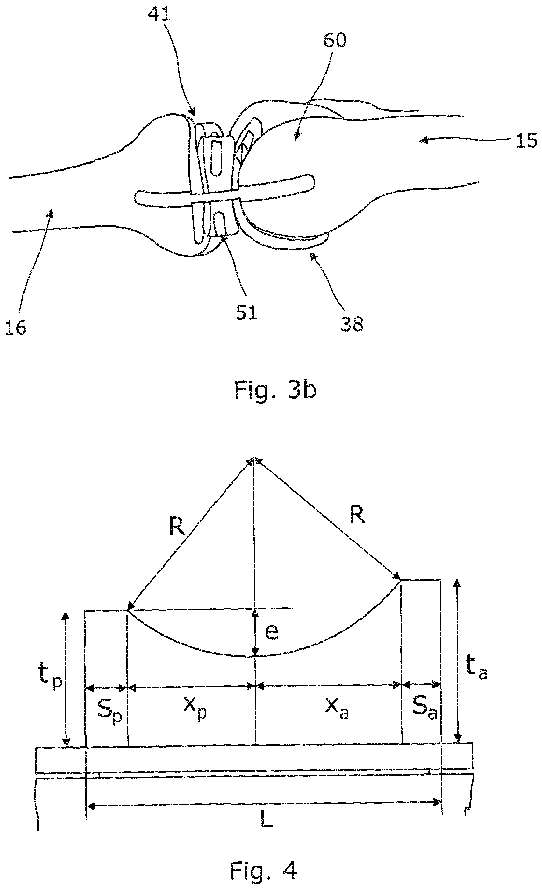

Figures 3a and 3b show the trial fitting of a trial meniscal bearing in a surgical operation. People come in different sizes, and prosthetic knee components come in different sizes, requiring femoral components of different radii & tibial components with different areas in plan view. After removing bone from a patient's damaged medial femoral condyle and fitting the femoral component 22 to it, and after removing bone from the damaged tibia and fitting the tibial component or plate 20 to it, there is a gap between the upper surface of the tibial plate and the domed surface of the femoral component. The meniscal bearing 24 is to fit in that gap.

The surgeon positions a patient's leg to a desired position, e.g. with the femur and tibia at about 90° to each other. The surgeon then selects a trial bearing 30, and inserts it into the gap to test the fit. The surgeon articulates the knee joint with the trial bearing in place to see if it will move properly, without problems, under a range of movement. There should still be a gap between the trial bearing and the other joint components, and that gap should remain more or less constant through the range of movement of the knee. The surgeon may choose to try a thicker bearing if he thinks that there is too much slack in the knee, or a thinner bearing if there is not enough slack.

The medial ligaments of the knee are strong and thick. It is hard to distract the joint (push the joint open) against their resistance. A surgeon often wants to ensure that the joint is not loose and so often tries to push into the gap the thickest meniscal bearing possible.

This can result in accidentally overstuffing the knee joint, which can cause problems later in use of the knee. The present invention helps to avoid such problems, especially with less experienced surgeons.

Up until now the entrapment of meniscal bearings has varied from about 3mm for small patients, to about 4mm for very large patients.

Previous bearings used in unicondylar knee replacements, as shown in figure 4, have been constructed with a posterior lip or projecting edge having a bearing thickness of between 3mm and 4mm above the base of the bowl or dish of the meniscal bearing. The anterior lip or projecting edge of the bearing has had a thickness of around 5mm above the base of the bowl. We have determined that, by reducing the entrapment to a range of about 3.2mm for very small patients to about 3.8mm for very large patients, or perhaps maintaining the entrapment of the meniscal bearing at around 3.5mm for all sizes, it is more difficult to overstuff the joint in small patients, assisting in reducing post-operative pain to the patient, and it is easier to implant the correct thickness of bearing in large patients.

In our bearing we have realised that we may want consistent entrapment for any bearing thickness at its centre. That is to say, our bearing will sit with about the same level of slackness once it is in situ, and therefore we will avoid (or reduce the chance of) overstuffing. In the prior art the entrapment ($t_{max}$-$t_{min}$) is normally about 3mm to about 4mm for the range of sizes of bearings. We would have consistent entrapment of about 3.5mm, or a reduced entrapment range of 3.2mm to 3.8mm, increasing entrapment in the smaller sizes and decreasing it in the larger ones.

Table 1 below shows the relationship between $t_{max}$ and $t_{min}$ for some of the known prior art medial unicondylar meniscal bearings. In these cases, the anterior and posterior thicknesses are equal:

**Table 1 (Extra. Large Bearings)**

| Size | $t_{max}$ (mm) ± 0.25 | $t_{min}$ (mm) ± 0.25 | $t_{max}$ - $t_{min}$ | anterior-posterior length L (mm) ± 0.5 |
|------|------|------|------|------|
| 3XL | 7.54 | 3.5 | 4.04 | 39.5 |
| 4XL | 8.54 | 4.5 | 4.04 | 39.5 |
| 5XL | 9.54 | 5.5 | 4.04 | 39.5 |
| 6XL | 10.54 | 6.5 | 4.04 | 39.5 |
| 7XL | 11.54 | 7.5 | 4.04 | 39.5 |
| 8XL | 12.54 | 8.5 | 4.04 | 39.5 |
| 9XL | 13.54 | 9.5 | 4.04 | 39.5 |

In a large prior art bearing the sizes are shown in Table 2:

**Table 2**

| Size | $t_{max}$(mm) ± 0.25 | $t_{min}$ (mm) ± 0.25 | $t_{max}$ $t_{min}$ | anterior-posterior length L (mm) ± 0.5 |
|------|------|------|------|------|
| 3L | 7.26 | 3.5 | 3.76 | 36.8 |
| 4L | 8.26 | 4.5 | 3.76 | 36.8 |
| 5L | 9.26 | 5.5 | 3.76 | 36.8 |

(continued)

| Size | $t_{max}$ (mm) ± 0.25 | $t_{min}$ (mm) ± 0.25 | $t_{max}$ $t_{min}$ | anterior-posterior length L (mm) ± 0.5 |
|---|---|---|---|---|
| 6L | 10.26 | 6.5 | 3.76 | 36.8 |
| 7L | 11.26 | 7.5 | 3.76 | 36.8 |
| 8L | 12.26 | 8.5 | 3.76 | 36.8 |
| 9L | 13.26 | 9.5 | 3.76 | 36.8 |

In a medium prior art bearing the sizes are as shown in Table 3:

**Table 3**

| Size | $t_{max}$ (mm) ± 0.25 | $t_{min}$ (mm) ± 0.25 | $t_{max}$ - $t_{min}$ | anterior-posterior length L (mm) ± 0.5 |
|---|---|---|---|---|
| 3M | 7.0 | 3.5 | 3.5 | 34.2 |
| 4M | 8.0 | 4.5 | 3.5 | 34.2 |
| 5M | 9.0 | 5.5 | 3.5 | 34.2 |
| 6M | 10.0 | 6.5 | 3.5 | 34.2 |
| 7M | 11.0 | 7.5 | 3.5 | 34.2 |
| 8M | 12.0 | 8.5 | 3.5 | 34.2 |
| 9M | 13.0 | 9.5 | 3.5 | 34.2 |

In a prior art small bearing the sizes are as shown in Table 4:

**Table 4**

| Size | $t_{max}$ (mm) ± 0.25 | $t_{min}$ (mm) ± 0.25 | $t_{max}$ - $t_{min}$ | anterior-posterior length L (mm) ± 0.5 |
|---|---|---|---|---|
| 3S | 6.73 | 3.5 | 3.23 | 31.6 |
| 4S | 7.73 | 4.5 | 3.23 | 31.6 |
| 5S | 8.73 | 5.5 | 3.23 | 31.6 |
| 6S | 9.73 | 6.5 | 3.23 | 31.6 |
| 7S | 10.73 | 7.5 | 3.23 | 31.6 |
| 8S | 11.73 | 8.5 | 3.23 | 31.6 |
| 9S | 12.73 | 9.5 | 3.23 | 31.6 |

In an extra small prior art bearing the sizes are as shown in Table 5:

**Table 5**

| Size | $t_{max}$ (mm) ± 0.25 | $t_{min}$ (mm) ± 0.25 | $t_{max}$ - $t_{min}$ | anterior-posterior length L (mm) ± 0.5 |
|---|---|---|---|---|
| 3XS | 6.48 | 3.5 | 2.98 | 29.2 |
| 4XS | 7.48 | 4.5 | 2.98 | 29.2 |
| 5XS | 8.48 | 5.5 | 2.98 | 29.2 |
| 6XS | 9.48 | 6.5 | 2.98 | 29.2 |
| 7XS | 10.48 | 7.5 | 2.98 | 29.2 |
| 8XS | 11.48 | 8.5 | 2.98 | 29.2 |
| 9XS | 12.48 | 9.5 | 2.98 | 29.2 |

Figure 2d shows a prior art meniscal bearing for a medial unicondylar arthroplasty. The height $t_p$ is lower than

the height $t_a$: that is to say the bearing has a thicker dimension at its anterior end than at its posterior end. As discussed in relation to Tables 1 to 5, the entrapment at its posterior end ranges from about 4mm for the extra large bearing range to 3mm in the extra small size range.

We have discovered that there may be advantages in having the entrapment proportionally larger in smaller patients and proportionally smaller in larger patients.

It will be appreciated that smaller people need smaller prosthetic components, including smaller bearings. It is well known to have several sizes of femoral, tibial and bearing components. Each patient is adjudged by a medical practitioner (possibly the surgeon in a pre-operative review) to be extra small, small, medium, large, or extra large. At the time of surgery the surgeon is provided with a kit of trial components that are "extra small", "small", "medium", "large", or "extra large". In each kit there is a range of different sizes of trial tibial components, a range of different sizes of trial femoral components and a range of different sizes of trial meniscal bearings.

It will be appreciated that the surgeon is in the operating theatre with the patient and, e.g., 6 or 7 trial tibial components, 6 or 7 trial femoral components, and 6 or 7 trial sets of meniscal bearings. As he selects/tries out the trial components he selects the correct size to be used. The implantable prosthetic components corresponding to the trial size are then ordered and delivered, typically from a store outside of the operating theatre, and the implantable components are fitted into place.

The surgeon may first remove a slice of bone from the medial tibial plateau, exposing a plane surface. He selects the correct size of tibial template, a plate with flat upper and lower surfaces, which best fits the exposed surface. He removes a sliver of bone from the posterior femoral condyle. He confirms that the femoral and tibial components are correctly placed so that the minimum gap between the components in extension is the same as that in flexion. The surgeon then has to select the correct thickness of bearing.

After the surgeon has fitted the femoral component (having first tried selected trial components and having selected the size that best suits the patient after femoral bone has been removed), and after fitting the tibial component (having first tried selected trial components to find the size of tibial component that best suits the patient after tibial bone has been removed), the surgeon has to select the correct thickness of bearing.

The surgeon has a range of, for example, large left leg medial trial meniscal bearings to choose from, each with an associated cylindrical gap gauge. He inserts a gap gauge into the gap between the tibial and femoral components and flexes and extends the knee. He progressively removes bone from the damaged distal femoral condyle until the minimum gap between the components in extension is the same as that in flexion. If the gap gauge seems too loose he takes it out and inserts a thicker gap gauge. He confirms his choice of bearing thickness by inserting the corresponding trial bearing. Traditionally, surgeons are afraid of having the joint too loose and so they tend to choose the thickest size bearing they can force in. This can result in overstuffing.

We have realised that in the prior art, the lower/thinner posterior end of the bearing also means that the entrapment to the rear of the bearing is lower than the entrapment to the front of the bearing (see Figure 2d).

In our invention, the entrapment at the rear may be larger than in the prior art for small and extra small bearings, and smaller than in the prior art for large and extra large bearings. The entrapment at the rear may be around 3.5mm for all bearing sizes in our invention.

By entrapment, we may mean the difference in thickness between the lowermost portion of the dished surface of the bearing and whichever is the thinnest/shallowest of the anterior or posterior end regions of the bearing if they have different thicknesses.

The anterior-posterior length of the bearing varies between sizes of bearing (e.g. between extra small, small, medium, large and extra large).

For a part spherical bearing surface, Figure 4 illustrates that the entrapment is related to the radius of curvature R of the part spherical surface and the horizontal distance $x_p$ from the posterior extreme of the curved surface to the lower extreme of the curved surface.

The length of the bearing L is determined by the size of the patient's bone structure - it should not be too long. The distance $s_a$ and $s_p$ from the anterior and posterior extremities of the bearing respectively to the start of the part-spherical bearing surface are shown in Figure 4. In some embodiments $s_a = s_p$, but in others it does not.

As shown in Figure 4, by Pythagoras' Theorem,

$$e = R - \sqrt{R^2 - x_p{}^2}$$

or expressed another way:

$$x_p = \sqrt{2Re - e^2}$$

where e = the entrapment, R= the radius of curvature, and x = the length of the chord from the vertical centre line to the posterior of the curved bearing surface.

If $s_a = s_p$ = about 4mm, e = 3.5mm and the anterior-posterior bearings are as in Tables 1 to 5 of the prior art, then this gives:-

| Size of Bearing | R | $x_p$ |
| --- | --- | --- |
| Extra large | 27.92 | 13.53 |
| Large | 26.08 | 13.05 |
| Medium | 24.25 | 12.55 |
| Small | 22.35 | 12.01 |
| Extra small | 20.7 | 11.5 |

We have appreciated that for some of our embodiments, instead of, as in the prior art, having the posterior end of the bearing less thick than the anterior end - making it easier to push in, we should have the posterior end not substantially thinner, or no thinner, than the anterior end (and in some embodiments the same thickness).

This will, for the same anterior thickness, make the bearing harder to push in. This reduces the chance of overstuffing - any given level of force to push the test bearing results in the choice of a final bearing that is a little thinner at the anterior end than was previously the case, and so for the same sized (length anterior-posterior and radius of curvature R) bearing we have a similar entrapment, and, compared to the prior art, a thinner (as measured at the depth of the spherical socket) bearing, but a thicker bearing, as measured at the posterior end, that is harder to push in.

In some embodiments, our bearing is symmetric about a coronal plane and also about a sagittal central plane. This means that our bearing is no longer handed - we no longer need a left knee medial bearing and a different, handed, right knee medial bearing. Similarly, our test bearings need then not be handed - they could be symmetric. This can reduce parts inventory. They can also be inserted either way around. They do not have different anterior and posterior profiles and heights: they are the same.

In another embodiment, our bearing is implanted by holding it against the anterior surface of the femoral component and sliding it round into the gap between the femoral and tibial components. This may require smaller distraction of the femoral component and a smaller force required for distraction for a given entrapment. This is true for a smaller measurement of $s_p$ from the posterior extremity of the bearing to the start of the part-spherical bearing surface.

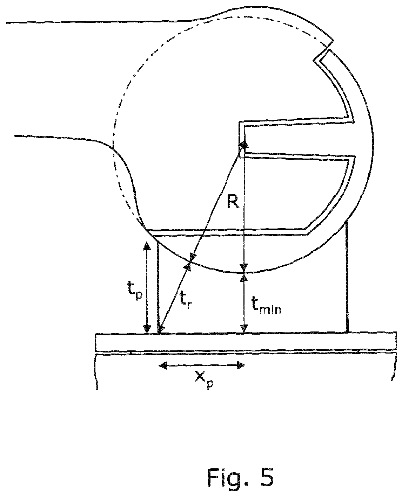

Figure 5 shows a unicondylar knee replacement including a bearing with negligible values of $s_a$ and $s_p$.

The vertical thickness of the posterior extremity of the bearing $t_p$ is given by

$$t_p = t_{min} + R - \sqrt{R^2 - x_p^2}$$

R is the external radius of the femoral component and the radius of the concavity of the upper surface of the bearing and $x_p$ is the posterior half length of the concavity of the upper surface of the bearing.

$t_{min}$ is minimum thickness of the meniscal bearing.

For a given $t_{min}$ + R, $t_p$ can be increased by increasing $x_p$

The maximum radial thickness of the posterior end of the bearing $t_r$ is given by

$$t_r = \sqrt{(R + t_{min})^2 + x_p^2} - R$$

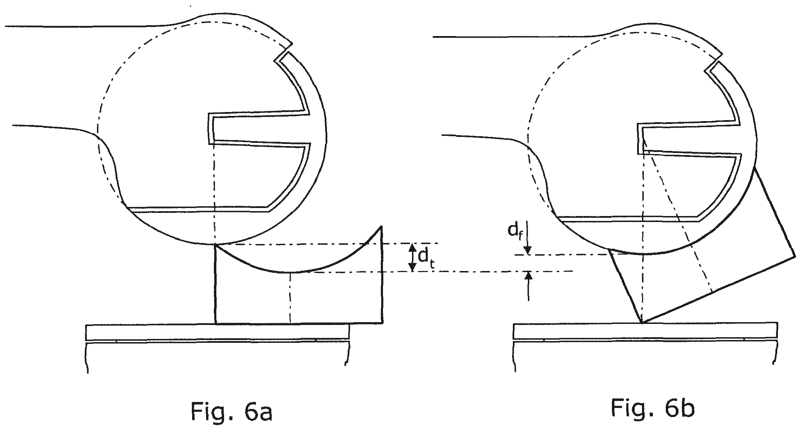

For negligible values of $s_a$, the vertical thickness of the posterior extremity $t_p$ will always be larger than the maximum radial thickness $t_r$. Therefore less distraction and force will be required when implanting the bearing along the femoral component, as shown in figure 6b.

The distraction $d_f$ required for implantation of the bearing along the femoral component, as shown in figure 6b, is as follows:

$$d_f = t_r - t_{min} = \sqrt{(R + t_{min})^2 + x_p^2} - R - t_{min}$$

From this, it can be deduced that, as the minimum thickness of the bearing $t_{min}$ is increased, the distraction, and therefore the force, required for implantation along the femur decreases.

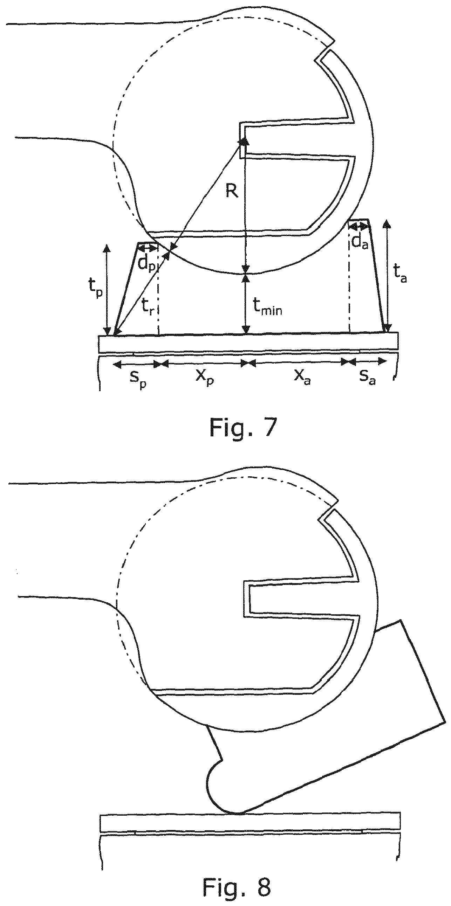

In some embodiments, the distraction and force required to insert the bearing horizontally along the tibia is equal to the distraction required to insert the bearing along the femur. Using the geometry in figure 7, it can be shown that the vertical thickness of the posterior extremity will be equal to the radial thickness when $s_p$ is calculated as follows:

*For*

$$t_p = t_r, \qquad s_p = \sqrt{\left(t_{min} + 2R - \sqrt{R^2 - x_p^2}\right)^2 - (t_{min} + R)^2 - x_p}$$

| Size of Bearing | $x_p$ | R | $s_p$ |
|---|---|---|---|
| Extra large | 15.5 | 32 | 1.9 |
| Large | 14 | 26.5 | 2.1 |
| Medium | 13 | 23 | 2.3 |
| Small | 11.5 | 18.5 | 2.5 |
| Extra small | 10.5 | 16 | 2.6 |

In some embodiments, the distraction and force required to insert the bearing horizontally along the tibia are larger than the distraction and force required to insert the bearing along the femur. This will be the case when $s_p$ is larger than the corresponding value given above.

In some embodiments, the distraction and force required to insert the bearing horizontally along the tibia is larger than the force and distraction required to insert the bearing along the femur. This will be the case when $s_p$ is smaller than the corresponding value given above.

In order to increase the posterior half-length of the bearing while not significantly increasing the minimum posterior radial thickness of the bearing, material can be added to the posterior vertical surface in a semi-cylindrical shape, as shown in figure 8, to form a protrusion.

**Claims**

1. A system of parts for use in unicondylar meniscal bearing knee replacement, the system comprising;
   a plurality of meniscal bearings ranging in size including an extra small bearing having an anterior-posterior length of 29.2±0.5mm, a small bearing, a medium bearing, a large bearing, and an extra large bearing having an anterior-posterior length of 39.5±0.5mm, each meniscal bearing comprising a body defining a dished first bearing surface on one side thereof and a second surface on an opposing side thereof;
   wherein each meniscal bearing has an entrapment defined as the difference in thickness between a lowermost portion of the bearing and the thinnest of either an anterior thickness or a posterior thickness of the bearing , the entrapment of each meniscal bearing being between 3.2mm at least for the extra small bearing and 3.8mm at most for the extra large bearing.

2. The system of claim 1, wherein the entrapments of the meniscal bearings are all approximately the same.

3. The system of claim 2, wherein the entrapments of the meniscal bearings are all approximately 3.5mm.

4. The system of claim 1, wherein the meniscal bearings have a plurality of different lengths.

5. The system of claim 1, wherein the length of at least one of the meniscal bearings is at least 39mm.

6. The system of claim 1, wherein the length of at least one of the meniscal bearings is less than 31.6mm.

7. The system of claim 1, wherein the meniscal bearings have a plurality of different minimum thicknesses between the first and second surfaces.

8. The system of claim 1, wherein the meniscal bearings of a given length have approximately the same entrapment.

9. The system of claim 1, wherein the meniscal bearings are trial bearings for use in fitting a prosthesis or implantable bearings for use with the prosthesis.

10. The system of claim 1, further comprising;
a set of trial bearings and a set of implantable bearings, each implantable bearing corresponding to one of the trial bearings.

11. A kit of parts for use in unicondlyar meniscal bearing knee replacement comprising a plurality of meniscal bearings, each meniscal bearing comprising a body defining a dished first bearing surface on one side thereof and a second surface on an opposing side of the body, in which each meniscal bearing has an entrapment, the entrapment of each meniscal bearing being between 3.2mm and 3.8mm, and wherein each meniscal bearing has a length from an anterior end to a posterior end with a length selected from a plurality of different lengths, the length of at least one of the meniscal bearings being at least 39mm or at least 39.4mm

12. The kit of claim 11, wherein each meniscal bearing has a length selected from a plurality of different lengths comprising 29.2mm (extra small), 31.6mm (small), 34.2mm (medium), 36.8mm (large) and 39.5mm (extra large).

13. A kit of parts for use in unicondylar meniscal bearing knee replacement, comprising a plurality of meniscal bearings, each meniscal bearing comprising a body defining a dished first bearing surface on one side thereof and a second surface on an opposing side of the body, in which each meniscal bearing has an entrapment, the entrapment of each meniscal bearing being between 3.2mm and 3.8mm.

14. The kit of claim 13, in which the entrapments of the meniscal bearings are all approximately the same.

15. The kit of claim 14, in which the entrapments are all approximately 3.5mm.

16. The kit of any preceding claim, in which each meniscal bearing has a length from an anterior end to a posterior end, the meniscal bearings having a plurality of different lengths.

17. The kit of claim 4, in which the length of at least one of the meniscal bearings is at least 39mm, or at least 39.4mm.

18. The kit of claim 16 or claim 17 in which the length of at least one of the meniscal bearings is less than 31.6mm or 29.5mm.

19. The kit of any preceding claim, in which the meniscal bearings have a plurality of different minimum thicknesses between the first and second surfaces.

20. The kit of claim 19, in which the meniscal bearings of a given length have approximately the same entrapment.

21. The kit of any preceding claim, in which the meniscal bearings are trial bearings for use in fitting a prosthesis, or implantable bearings for use with the prosthesis.

22. The kit of any preceding claim, comprising a set of trial bearings and a set of implantable bearings, each implantable bearing corresponding to a trial bearing.

23. The kit of claim 22, in which the correspondence between trial and implantable bearings is such that the surgeon fits the thickest grade trial bearing they can into the gap between the tibial and femoral components of a patient's knee, and then removes the trial bearing and selects the implantable bearing to be one grade thinner than the thickest trial bearing that fitted in the gap.

24. The kit of any preceding claim, in which at least one, or all, of the meniscal bearings are symmetrical about either or both of a coronal and a sagittal plane.

25. The kit of any preceding claim, comprising at least one femoral prosthetic component having a spherical articular surface having a radius of curvature, the kit comprising meniscal bearings in which the first surface has the same radius of curvature.

26. The kit of any preceding claim, comprising at least one tibial prosthetic component.

27. The kit of any preceding claim, in which at least one, or preferably all of, the meniscal bearings comprise a protrusion at a posterior end thereof, an anterior end thereof or both.

28. The kit of claim 27, in which the protrusion is of even depth along the end to which it is added.

29. The kit of claim 27, in which the protrusion has a reduced depth at the upper end of the surface compared with the lower end, giving a sloped edge.

30. A meniscal bearing for use in a unicondylar meniscal bearing knee replacement, comprising a body defining a dished first bearing surface on one side thereof and a second surface on an opposing side of the body, the meniscal bearing having a length from an anterior end to a posterior end being greater than 39mm or less than 31. 6mm, and having an entrapment being the maximum difference in thickness between the first and second surfaces of between 3.2mm and 3.8mm.

31. The meniscal bearing of claim 30, in which the length is greater than 39 .4mm, or less than 29. 5mm.

32. A method of performing a unicondylar meniscal bearing knee replacement, the method comprising the implantation of a femoral component into the femur of a patient, the implantation of a tibial component into the tibia of the patient, the selection of a meniscal bearing from the kit of claims 13 to 29, or of a meniscal bearing according to claims 30 or 31, and the insertion of the meniscal bearing between the femoral component and the tibial component.

33. The method of claim 32, comprising inserting the bearing at an angle to an articular surface of tibial component so that the first surface of the meniscal bearing substantially contacts a bearing surface of the femoral component throughout insertion of the meniscal bearing.

Fig. 1

Fig. 2a

$t_{max}$

$t_{min}$

48

50

46

Fig. 2b

56

56

52

62

60

54

58

Fig. 2c
(Prior Art)

Fig. 2d
(Prior Art)

Fig. 3a

41

60

15

16

51

38

Fig. 3b

$t_p$ $S_p$ $x_p$ $e$ $R$ $R$ $x_a$ $S_a$ $t_a$

$L$

Fig. 4

Fig. 5

Fig. 6a

Fig. 6b

EP 3 225 213 A1

Fig. 7

Fig. 8

Europäisches
Patentamt

European
Patent Office

Office européen
des brevets

# EUROPEAN SEARCH REPORT

**Application Number**

EP 16 18 5377

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2004/069089 A2 (AESCULAP AG & CO KG [DE]; REICH JAN [DE]) 19 August 2004 (2004-08-19) * figures 1,2 * | 1-33 | INV. A61F2/38 ADD. A61F2/30 |
| A | US 2006/190086 A1 (CLEMOW ALASTAIR J [US] ET AL) 24 August 2006 (2006-08-24) * figures 18,19,22 * | 1-33 | |
| A | WO 2004/006811 A2 (ADVANCED BIO SURFACES INC [US]; FELT JEFFREY C [US]; RYDELL MARK A [US]) 22 January 2004 (2004-01-22) * figures 1,2 * | 1-33 | |
| A | US 2009/259314 A1 (LINDER-GANZ ERAN [IL] ET AL) 15 October 2009 (2009-10-15) * the whole document * | 1-33 | |
| A | GB 1 534 263 A (NAT RES DEV) 29 November 1978 (1978-11-29) * the whole document * | 1-33 | TECHNICAL FIELDS SEARCHED (IPC) |
| A | US 6 206 927 B1 (FELL BARRY M [US] ET AL) 27 March 2001 (2001-03-27) * figure 5 * | 1-33 | A61F |

The present search report has been drawn up for all claims

| Place of search | Date of completion of the search | Examiner |
|---|---|---|
| Munich | 31 July 2017 | Cuiper, Ralf |

## ANNEX TO THE EUROPEAN SEARCH REPORT
## ON EUROPEAN PATENT APPLICATION NO.

EP 16 18 5377

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2017

| Patent document cited in search report | | Publication date | Patent family member(s) | | Publication date |
|---|---|---|---|---|---|
| WO 2004069089 | A2 | 19-08-2004 | DE | 10305795 A1 | 26-08-2004 |
| | | | DE | 20302179 U1 | 24-04-2003 |
| | | | WO | 2004069089 A2 | 19-08-2004 |
| US 2006190086 | A1 | 24-08-2006 | CA | 2598630 A1 | 31-08-2006 |
| | | | CN | 101160107 A | 09-04-2008 |
| | | | EP | 1850804 A1 | 07-11-2007 |
| | | | JP | 2008541785 A | 27-11-2008 |
| | | | US | 2006190086 A1 | 24-08-2006 |
| | | | WO | 2006091495 A1 | 31-08-2006 |
| WO 2004006811 | A2 | 22-01-2004 | AT | 398432 T | 15-07-2008 |
| | | | AU | 2003247952 A1 | 02-02-2004 |
| | | | CA | 2492030 A1 | 22-01-2004 |
| | | | EP | 1523291 A2 | 20-04-2005 |
| | | | ES | 2309350 T3 | 16-12-2008 |
| | | | US | 2006111726 A1 | 25-05-2006 |
| | | | WO | 2004006811 A2 | 22-01-2004 |
| US 2009259314 | A1 | 15-10-2009 | US | 2009259314 A1 | 15-10-2009 |
| | | | US | 2011288643 A1 | 24-11-2011 |
| | | | US | 2016235544 A1 | 18-08-2016 |
| GB 1534263 | A | 29-11-1978 | CH | 597847 A5 | 14-04-1978 |
| | | | DE | 2550704 A1 | 26-05-1976 |
| | | | FR | 2290883 A1 | 11-06-1976 |
| | | | GB | 1534263 A | 29-11-1978 |
| | | | IE | 42356 B1 | 30-07-1980 |
| | | | JP | S5841855 B2 | 14-09-1983 |
| | | | JP | S51116095 A | 13-10-1976 |
| US 6206927 | B1 | 27-03-2001 | NONE | | |

EPO FORM P0459

For more details about this annex : see Official Journal of the European Patent Office, No. 12/82